Europäisches Patentamt

European Patent Office

Office européen des brevets

(19)

(11) Numéro de publication : **0 165 172**
**B1**

(12)

# FASCICULE DE BREVET EUROPÉEN

(45) Date de publication du fascicule du brevet :
25.05.88

(51) Int. Cl.⁴ : **C 12 M 3/00, C 12 M 1/00**

(21) Numéro de dépôt : **85401107.9**

(22) Date de dépôt : **05.06.85**

(54) **Appareil modulaire pour la culture cellulaire.**

(30) Priorité : 06.06.84 FR 8408839

(43) Date de publication de la demande :
18.12.85 Bulletin 85/51

(45) Mention de la délivrance du brevet :
25.05.88 Bulletin 88/21

(84) Etats contractants désignés :
AT BE CH DE FR GB IT LI LU NL SE

(56) Documents cités :
DE-A- 2 633 085
DE-A- 2 945 339
FR-A- 1 577 539
GB-A-   926 541
GB-A- 1 112 919

(73) Titulaire : **Bisconte, Jean-Claude**
**35 rue du Professeur Pauchet**
**F-92420 Vaucresson (FR)**

(72) Inventeur : **Bisconte, Jean-Claude**
**35, rue du Professeur Pauchet**
**F-92420 Vaucresson (FR)**

(74) Mandataire : **Orès, Bernard et al**
**Cabinet ORES 6, Avenue de Messine**
**F-75008 Paris (FR)**

Jouve, 18, rue St-Denis, 75001 Paris, France

## Description

La présente invention est relative à un appareil modulaire permettant d'exécuter les phases essentielles de la culture et de l'analyse cellulaire en faisant appel, éventuellement, à des automates.

La culture de cellules procaryotes et eucaryotes est pratiquée dans les laboratoires et les industries des domaines principaux suivants :

recherche fondamentale et appliquée concernant les mécanismes cellulaires (croissance, différenciation, interaction) dans des disciplines variées (biologie cellulaire, cancérologie, hormonologie, étude et tests de médicaments, etc...) ;

recherche et industrie de la biotechnologie en vue de la manipulation et de la sélection cellulaire (clonage d'hybridomes, par exemple) ;

analyse toxicologique d'aliments, d'effluents, de colorants ou autres, et

diagnostic médical passant par la préparation de cultures humaines (cytogénétique, cancérologie) ou bactériennes.

La culture cellulaire fait généralement appel à deux catégories de méthodes, selon la finalité poursuivie :

d'une part, les méthodes manuelles, universellement employées à des fins préparatoires et analytiques à petite échelle, dans lesquelles on dispose des matériels principaux suivants :

enceintes thermostatées (à $CO_2$ ou non),

hottes à flux laminaire,

récipients de culture constitués par des boîtes de Petri, des boîtes multitrous ou des flacons,

les mises en culture ou les prélèvements de cellules ou de milieu nutritif se faisant manuellement à la pipette Pasteur ou, plus récemment, à l'aide de dispositifs semi-automatisés à cônes plastiques jetables, tandis que l'analyse morphologique se fait généralement manuellement sous microscope inversé,

d'autre part, les méthodes automatisées dans des bioréacteurs qui permettent la culture en masse de cellules flottantes à des fins de production industrielle et qui ne sont pas directement concernées par la présente invention.

Rappelons que les méthodes manuelles comprennent les phases principales suivantes :

1. prélèvement cellulaire (par dissociation, repiquage) ;

2. mise en culture ;

3. entretien de la culture, en vue d'obtenir notamment la croissance et la différenciation, ce qui nécessite :

le stockage dans des conditions définies en température, hygrométrie, % $CO_2$ et pH,

le renouvellement du milieu nutritif, et

le contrôle de non-contamination et de développement du processus ;

4. l'interaction avec la culture cellulaire, par exemple par addition d'un produit approprié ou de cellules ;

5. le prélèvement de cellules en vue de repiquage, ou le prélèvement du milieu nutritif en vue de dosages, et

6. l'analyse morphologique, biochimique, physique ou autre de la culture cellulaire.

Les phases susdites constituent en réalité une séquence d'opérations dissociées qui, dans tous les cas, conduit à des variations momentanées de conditions dont il est difficile d'apprécier toutes les conséquences. En fait, on fait transiter lesdits récipients de culture entre des appareils différents :

étuve de stockage des récipients de culture,

hotte à flux laminaire pour l'exécution des manipulations,

observation microscopique dans un but de contrôle ou d'analyse, ce qui conduit au minimum à une augmentation des risques de contamination réciproque (homme — cellules en culture) et conduit aussi à un choc thermique qui perturbe le métabolisme cellulaire.

De plus, le nombre élevé de récipients de culture à entretenir, l'exigence de les manipuler le moins possible et l'absence de moyens adaptés interdisent d'utiliser toutes les informations contenues dans les cultures.

En résumé, les problèmes principaux que posent les méthodes manuelles actuellement utilisées consistent dans :

des risques élevés de contamination à l'encontre des cellules cultivées (eucaryotes surtout), à l'encontre des personnels si les cellules sont elles-mêmes pathogènes ou si les manipulations mettent en oeuvre des agents dangereux (cancérogènes, virus, etc...) ;

des besoins importants en personnels, pour l'exécution d'un travail généralement fastidieux visant à l'entretien régulier des cultures cellulaires, ce qui entraîne des pertes de temps élevées liées à la dispersion des postes de travail et la difficulté d'optimiser les opérations ;

des coûts élevés en pièces spécialisées, en produits consommables (récipients de culture, milieux nutritifs, etc...) et en équipements, qu'il faut multiplier pour réduire les risques de contamination et pour tenir compte de la spécialisation des applications ;

une exploitation biologique difficile, notamment liée à la variabilité inévitable de la pratique humaine, aux conditions d'environnement non optimales (chocs thermiques, mécaniques et chimiques induits par les manipulations), l'impossibilité de suivre et de quantifier les processus biologiques qui se déroulent en permanence dans les cultures et a fortiori d'agir rétroactivement sur eux.

Toutefois, des améliorations ont été apportées dans certains domaines. C'est ainsi que :

l'étuve de stockage des cultures et la hotte à flux laminaire ont été juxtaposées par la Société HERAEUS ;

il existe des dispositifs nombreux de prélèvement ou de dilution du milieu cellulaire semi-automatisés ;

des Constructeurs comme LEITZ proposent des microscopes contenus dans des enceintes simplement thermostatées et à $CO_2$ mais ne réalisant pas la stérilité ni la constance d'un degré hygrométrique élevé, ce qui s'explique pour ce dernier point par l'incompatibilité avec les composants optiques et électromécaniques ;

l'inventeur a, pour sa part, proposé un dispositif associant la culture cellulaire à l'analyse d'image automatique en temps réel et en continu pour suivre les événements d'interaction cellulaire (MIKROSKOPIE, 1980) ;

d'autres solutions ont été proposées, toujours dans le sens d'une automatisation, comme celles de WALKER et POZNANOVIC (International Biotechnology Laboratory, Décembre 1983) qui ont réalisé un dispositif multichambres dans lequel les conditions sont programmables et s'applique particulièrement aux cellules en suspension, mais ce dispositif n'est pas approprié à :

la culture automatisée d'un nombre élevé d'échantillons,

l'usage de récipients existants dans le commerce,

l'analyse morphologique,

le clonage automatique, et se présente donc comme un dispositif très différent de celui que nous proposons.

On connaît également la Demande de Brevet DE-2 633 085 qui décrit un dispositif pour la culture automatique de tissus vivants ou de cellules vivantes, qui sont contenus dans des récipients de culture, conjointement avec une solution nutritive.

Ce dispositif comporte, en combinaison :

une enceinte étanche aux gaz, dans laquelle est disposée une pluralité de récipients de culture parmi lesquels se trouve un récipient contenant les tissus ou les cellules et la solution nutritive précités, de même que des récipients de culture vides,

un dispositif d'analyse pour l'analyse (ou l'observation) de la prolifération cellulaire des tissus ou des cellules contenus dans le récipient de cultures,

un dispositif qui est disposé à l'intérieur de l'enceinte, destiné à la préparation de subcultures, se composant de tissus ou cellules, par remplissage des récipients de culture vides avec les cellules en cours de séparation et déjà séparées, ainsi que d'une solution nutritive fraîche,

un dispositif de régulation des paramètres de l'atmosphère existant à l'intérieur de l'enceinte, pour les maintenir constants, et

un dispositif de manœuvre du dispositif d'analyse précité, du dispositif de remplissage, ainsi que du dispositif de régulation.

Les récipients de culture sont stockés sur un disque de support qui est susceptible d'être mis en rotation.

Le but de l'invention est d'éliminer les inconvénients des méthodes antérieures de culture cellulaire, en particulier en ce qui concerne les risques de contamination réciproque, les coûts d'investissement et les coûts de fonctionnement.

Un autre but de l'invention est d'intégrer toutes les phases liées à la culture cellulaire et qui, jusqu'à présent, constituent une séquence d'opérations dissociées, tout en pouvant faire appel à des récipients de culture traditionnels que l'on peut rendre réutilisables, sans pour cela exclure l'emploi de récipients spéciaux.

Un autre but de l'invention est de se rapprocher de conditions optimales en supprimant totalement les chocs thermiques, ainsi que mécaniques et chimiques, en réalisant des conditions de culture uniformes indépendamment de l'opérateur.

Un autre but de l'invention est de permettre d'asservir par des logiciels les conditions d'environnement des cellules, y compris le milieu nutritif, à des paramètres mesurés fréquemment ou même en continu.

La présente invention a pour objet un appareil modulaire de culture cellulaire, comprenant, dans une même enceinte étanche et à parois isothermes, en combinaison :

au moins un dispositif de stockage de récipients de culture, notamment constitués par des boîtes multitrous ou des flacons,

un dispositif de régulation de température,

un dispositif de stérilisation par flux laminaire filtré, en circuit fermé,

un dispositif d'alimentation et de régulation de gaz, tels que de l'air, du $CO_2$ et de la vapeur,

au moins un dispositif permettant d'effectuer une opération appropriée sur une culture cellulaire,

lequel appareil est caractérisé en ce que ledit dispositif de stockage de récipients de culture est un dispositif modulaire qui comporte une pluralité de modules constituant des rayonnages pourvus de glissières, le long desquelles coulissent des compartiments étanches, contenant des récipients de culture et obturés par des couvercles, notamment légèrement coniques, qui sont équipés d'une poignée et de joints toriques d'étanchéité.

Selon un mode de réalisation préféré de l'appareil conforme à l'invention, ladite enceinte étanche contient également au moins un bras automatique et programmable, qui est pourvu d'une pince de manipulation qui effectue toutes les opérations correspondant à l'emplacement de chaque dispositif permettant d'effectuer une opération appropriée sur les cultures cellulaires et définissant un poste de travail modulaire choisi en fonction des besoins, et un micro-ordinateur de pilotage de toutes les opérations suivant un protocole d'expériences classiques, ainsi que spécialement définies en fonction des besoins, lequel micro-ordinateur assure également le traitement des données.

Selon un mode de réalisation avantageux de l'appareil conforme à l'invention, chaque compartiment est tenu en place par l'intermédiaire d'une baguette anti-retour s'interposant entre la paroi du module de rayonnage et celle du compartiment lui-même.

Selon un autre mode de réalisation avantageux de l'appareil conforme à l'invention, la liaison entre modules de rayonnage contigus se fait par l'intermédiaire de barres verticales, notamment en forme de T, qui sont pourvues de tétons d'accrochage pénétrant dans des logements correspondants ménagés dans des ailettes latérales faisant saillie à partir des côtés verticaux postérieurs de chaque module de rayonnage.

Selon un autre mode de réalisation avantageux de l'appareil conforme à l'invention, chaque compartiment est transparent et un espace approprié est ménagé au-dessus et au-dessous de chaque compartiment de manière à introduire, à l'aide dudit bras manipulateur, un dispositif optique permettant d'effectuer des mesures appropriées, notamment de colorimétrie et d'opacité.

Selon un mode de réalisation préféré de l'appareil conforme à l'invention, l'alimentation en gaz de chaque compartiment se fait automatiquement à l'aide du bras manipulateur, qui aspire le gaz à renouveler, ce qui permet d'en analyser la composition, et qui injecte le gaz de renouvellement, à travers un système à deux clapets ménagés dans la poignée du couvercle d'obturation de chaque compartiment et pourvus de deux conduits communiquant avec un conduit d'injection et un conduit d'aspiration, respectivement, portés par le bras manipulateur et activés lors de la préhension de la poignée du couvercle par la pince du bras.

Selon une variante avantageuse de l'appareil conforme à l'invention, le dispositif de stockage est caractérisé par une pluralité de modules constituant des étuves à parois isothermes et à compartiments étanches et comprenant trois zones principales, à savoir :

une première zone, disposée à la partie avant, rassemblant les récipients de cultures dans lesdits compartiments,

une deuxième zone, disposée à la partie médiane, rassemblant les raccordements entre chaque compartiment et les différentes alimentations en gaz, et

une troisième zone, disposée à la partie arrière, rassemblant les dispositifs d'alimentation en gaz, les dispositifs de régulation et les filtres, lesquels compartiments sont pourvus d'un rebord sur leur face avant et sont appliqués, notamment par collage sur une plaque pourvue d'ouverture et fixée elle aussi — notamment par collage — sur le pourtour de la face avant de chaque étuve, lesdits compartiments étant en outre disposés suivant des rangées verticales et ayant des dimensions variées.

Selon un mode de réalisation avantageux de cette variante, l'alimentation en gaz de chaque compartiment se fait automatiquement à l'aide d'ergots à billes permettant l'ouverture des vannes d'alimentation lors de la fermeture des compartiments, tandis que l'ouverture de ces derniers désactive les vannes d'alimentation et les récipients de cultures, notamment du type multitrous, sont rendus solidaires des couvercles des compartiments, qui constituent ainsi des boîtes spéciales à tiroir destinées à coopérer avec lesdits ergots d'activation de l'alimentation en gaz au moment de l'introduction de ces boîtes spéciales dans lesdits compartiments.

Selon un autre mode de réalisation avantageux de cette variante, lesdits dispositifs d'alimentation en gaz concourent dans une chambre de mélange où l'on règle la teneur en air, en $CO_2$ et en vapeur d'eau, notamment, à l'aide des dispositifs de réglage connus en eux-mêmes, chambre dans laquelle coulisse un piston dirigeant le mélange de gaz vers le ou les compartiments sélectionnés.

Selon un autre mode de réalisation avantageux de l'appareil conforme à l'invention, ainsi que de sa variante, lesdits postes de travail modulaire sont constitués par au moins l'un des dispositifs suivants :

un dispositif d'alimentation en milieu nutritif enrichi et d'évacuation du milieu appauvri,

et/ou un dispositif de rinçage et de stérilisation des récipients,

et/ou un dispositif de distribution de milieux nutritifs,

et/ou un dispositif de distribution de substances, notamment pharmacologiques,

et/ou un dispositif de distribution de cellules,

et/ou au moins un dispositif d'observation comprenant des optiques photoniques, notamment acoustiques, couplées ou non à des platines motorisées, ainsi qu'à un analyseur d'images, à un appareil photographique ou à un magnétoscope,

et/ou des dispositifs d'analyse physico-chimique,

et/ou des dispositifs pour repiquage,

et/ou des dispositifs de stockage variés,

et/ou des dispositifs de préparation de récipients de culture, notamment par pulvérisation, de substrats appropriés, tels que du collagène.

Selon un autre mode de réalisation avantageux de l'appareil conforme à l'invention, ainsi que de sa variante, ledit dispositif d'évacuation du milieu nutritif appauvri consiste en un dispositif d'aspiration comprenant un micro-moteur pas-à-pas qui actionne, par l'intermédiaire d'un système mécanique réducteur, un piston dans un cylindre fileté à sa base inférieure, autour de laquelle se visse un cône d'aspiration à extrémité effilée et biseautée, une membrane étant serrée entre ce cône et l'embase dudit cylindre et supportée par un joint torique qui assure en même temps l'étanchéité nécessaire.

Selon une disposition avantageuse de ce mode de réalisation, le cône est relié à deux conduits permettant l'arrivée de fluides de rinçage et de stérilisation de la partie interne du cône après l'opération d'aspiration et d'évacuation du milieu appauvri.

Selon une modalité avantageuse de cette disposition, l'évacuation du milieu aspiré se fait dans un puits ménagé dans la table de travail de ladite enceinte, lequel puits sert également de reposoir pour le dispositif d'aspiration et est pourvu d'une série de couronnes de conduits, décalés et inclinés dans le sens de l'évacuation, qui sont destinés au rinçage externe, à la stérilisation et au séchage.

Selon une autre disposition avantageuse de l'appareil conforme à l'invention, ledit dispositif d'aspiration coopère avec un dispositif d'alimentation en liquide pourvu d'un raccord auto-obturant en matière souple, notamment en élastomère, qui est soumis au flux laminaire existant à l'intérieur de l'enceinte et dans lequel est introduit ledit cône d'aspiration, tandis que le réservoir d'alimentation est disposé à l'extérieur de l'enceinte et en ambiance froide, l'injection de milieu nutritif liquide neuf ou enrichi se faisant par inversion du sens de rotation dudit moteur pas-à-pas.

Selon une variante avantageuse de cette disposition, l'injection de milieu nutritif enrichi se fait à l'aide d'un dispositif d'injection qui est indépendant dudit dispositif d'aspiration et qui comprend des flacons contenant les liquides à injecter à l'intérieur d'un bloc réfrigérant, ces liquides étant acheminés par un ou plusieurs tuyaux, notamment en élastomère, réunis en faisceau et enveloppés par une gaine isotherme faisant saillie de la base du bloc réfrigérant, le débit de chaque tuyau d'alimentation étant contrôlé par une vanne stérilisable fonctionnant en tout ou rien.

Selon une modalité avantageuse de cette variante de disposition, la partie terminale du faisceau de tuyau est pourvue d'une gaine de protection anti-contamination dans laquelle est acheminé un jet d'air permanent et à faible pression qui empêche le reflux des particules de liquide, cette gaine anti-contamination et les extrémités desdits tuyaux d'alimentation étant inclinés de façon à diriger le liquide d'injection contre la paroi desdites logettes de culture et à éviter l'impact direct contre les cellules qui adhèrent au fond de chaque logette.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

la figure 1 est une vue en perspective schématique qui montre un mode de réalisation de l'enceinte de l'appareil modulaire selon l'invention, dans laquelle sont visibles plusieurs dispositifs de stockage des cultures cellulaires conformes à l'invention (pour des raisons de simplification on n'a pas représenté les dispositifs de régulation thermique, de stérilisation et d'alimentation gazeuse ainsi que les différents dispositifs supplémentaires pouvant enrichir l'enceinte et constituant autant de postes de travail modulaires connus en eux-mêmes) ;

la figure 2 est une vue schématique en coupe transversale de l'appareil modulaire selon l'invention qui montre en particulier les dispositifs de régulation thermique et de stérilisation du flux laminaire, notamment vertical, qui ne sont pas représentés à la figure 1, ainsi qu'un bras manipulateur automatique pouvant également équiper l'enceinte ;

la figure 3 montre une vue en perspective d'un mode de réalisation du dispositif de stockage de récipients de culture conformément à l'invention ;

la figure 4 montre une vue en coupe longitudinale d'un compartiment étanche qui peut être glissé dans le dispositif de stockage représenté à la figure 3 et qui contient notamment une boîte de culture et le couvercle assurant l'étanchéité, conformément à l'invention ;

la figure 5 montre les détails d'un couvercle associé aux compartiments de figure 4 pouvant coopérer avec la pince d'un bras manipulateur automatique et programmable ;

les figures 6 et 7 se réfèrent à un autre mode de réalisation dudit dispositif de stockage et à une variante des récipients de culture qui peuvent être alimentés en gaz de la partie arrière au lieu de la partie avant ;

les figures 8 à 10 montrent le dispositif d'aspiration/injection du milieu nutritif selon l'invention, et

les figures 11 et 12 montrent le dispositif d'injection du milieu nutritif neuf destiné à coopérer avec le dispositif représenté aux figures 8 et 9 utilisé comme dispositif d'aspiration seulement (à savoir, sans coopérer avec le dispositif d'alimentation en liquide de la figure 10).

Ces dessins et les parties descriptives correspondantes illustrent l'objet de l'invention.

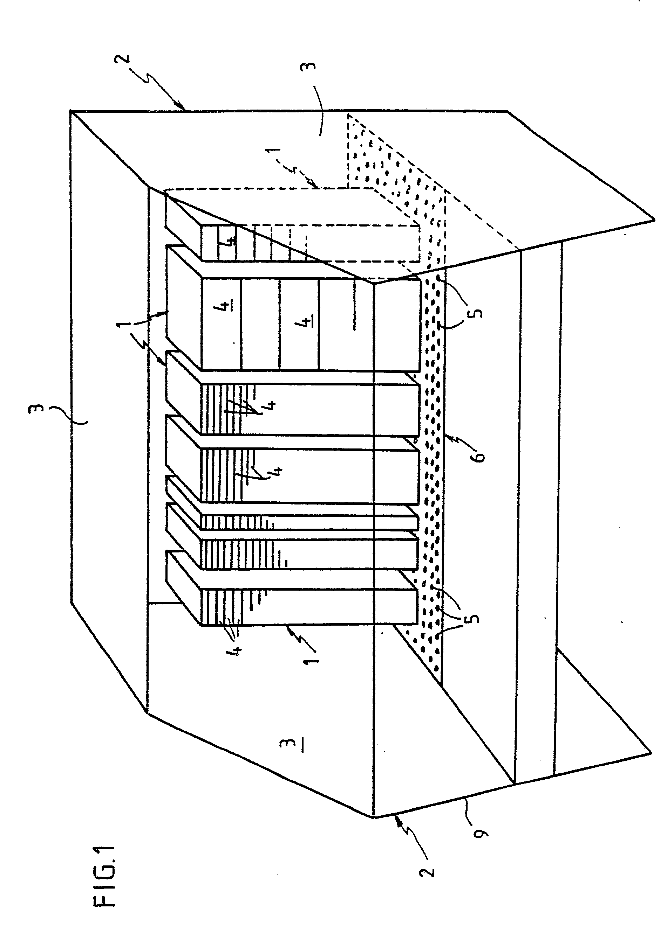

Les dispositifs de stockage 1, représentés schématiquement à la figure 1 et contenus dans une enceinte 2 dont les parois isothermes 3 sont de préférence en matière métallique ou plastique, notamment transparente en face avant, contiennent des récipients de culture 4 de dimensions variées.

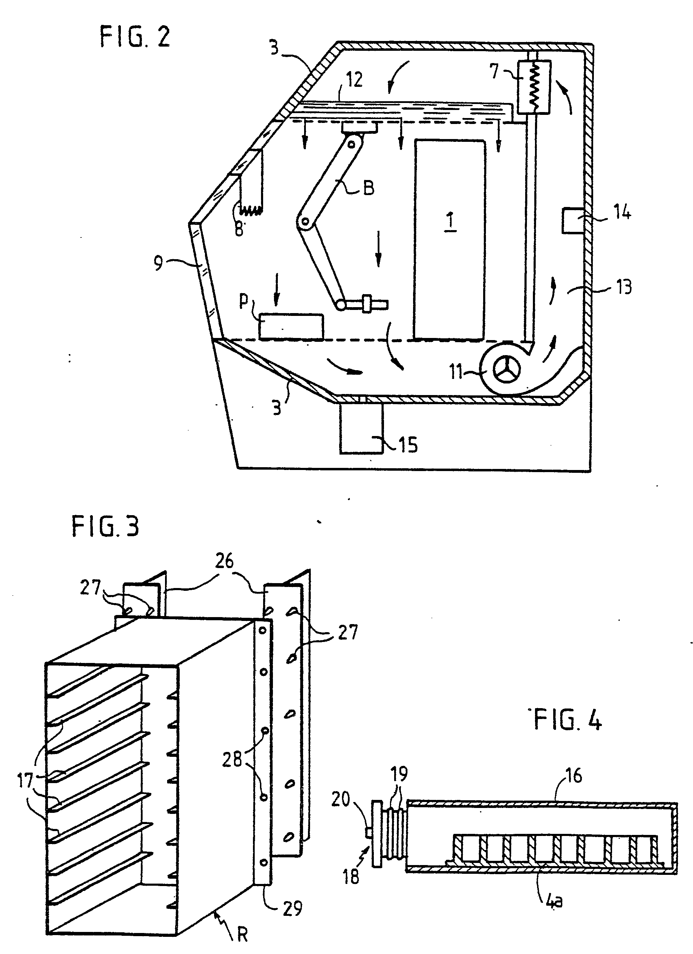

L'ensemble des postes de travail modulaires qui, comme précédemment décrit, peuvent éventuellement enrichir l'enceinte 2, en outre desdits dispositifs de stockage 1, au fur et à mesure de l'augmentation des besoins, est soumis à un flux laminaire stérile, en circuit fermé, qui de préférence est descendant (mais il pourrait être également ascendant ou horizontal), qui est repris par les perforations 5 de la table de travail 6 et qui est chauffé et régulé par le bloc de résistance 7 (cf. la figure 2). Les déperditions thermiques sont réduites par les parois isothermes 3, y compris la face avant de l'appareil qui est réalisée par exemple en double parois de verre ou de matière plastique. En fonctionnement normal l'enceinte 2 est étanche et l'accès manuel peut se faire par des passages équipés de gants de caoutchouc 8. Pour procéder à des manipulations, on peut également ouvrir le panneau vitré 9 ou utiliser un sas d'introduction (non représenté sur la figure 2). Le flux d'air chaud garantit ainsi des conditions thermiques homogènes dans tous les récipients, mais aussi sur le plateau de travail 6. Une turbine centrifuge 11 assure la circulation des gaz. La stérilité est assurée par la boîte à filtre 12.

Dans ces conditions, l'ensemble des dispositifs de stockage 1, est baigné par un flux stérile non turbulent qui tend à éliminer, de façon connue, d'éventuels germes de contamination. On peut d'ailleurs inclure dans le circuit de stérilisation par flux laminaire, et très avantageusement dans le circuit ascendant 13, un dispositif de stérilisation 14 par rayonnement ultraviolet.

La figure 2 montre également un bras manipulateur B qui peut éventuellement équiper l'enceinte 2 et qui est de préférence du type antropomorphique, mais qui peut être également du type à mouvement linéaire et à rotation.

En fonctionnement normal, lorsqu'un récipient de culture 4 est ouvert, tous les autres sont fermés, ce qui limite considérablement les risques de diffusion d'une contamination. Par ailleurs, une diffusion accidentelle de milieu nutritif liquide (pouvant éventuellement contenir des cellules) sur un poste de travail p, ne peut conduire à la diffusion d'une contamination qui en résulterait, parce qu'un dispositif 15 de recueil des liquides versés accidentellement sur le plateau de travail est associé à chaque poste p : en alternative, on peut utiliser un dispositif unique en forme d'entonnoir (non représenté) placé au-dessous de la table et pourvu d'un orifice latéral de communication avec le circuit 13. La figure 4 montre des récipients de culture, constitués en particulier par des boîtes multitrous 4a, qui sont introduits dans des compartiments 16 constitués très simplement par des boîtes à ouverture frontale réalisées par moulage, par exemple en matière plastique transparente telle que celle utilisée pour les récipients eux-mêmes.

Ces boîtes 16 sont empilées et collées, ou simplement placées, dans un dispositif de stockage constitué par exemple par un simple rayonnage R en métal (ou en matière plastique) qui se présente comme une boîte parallélépipédique ouverte sur la face avant et sur la face arrière et comportant des glissières 17 (cf. la figure 3). Ses dimensions dépendent de la taille des compartiments 16. Ces derniers (cf. la figure 4) comportent un couvercle équipé de joints toriques d'étanchéité 19. Ce couvercle est légèrement conique et est pourvu d'une poignée 20 pour faciliter son introduction à la main ou par ledit bras manipulateur automatique B.

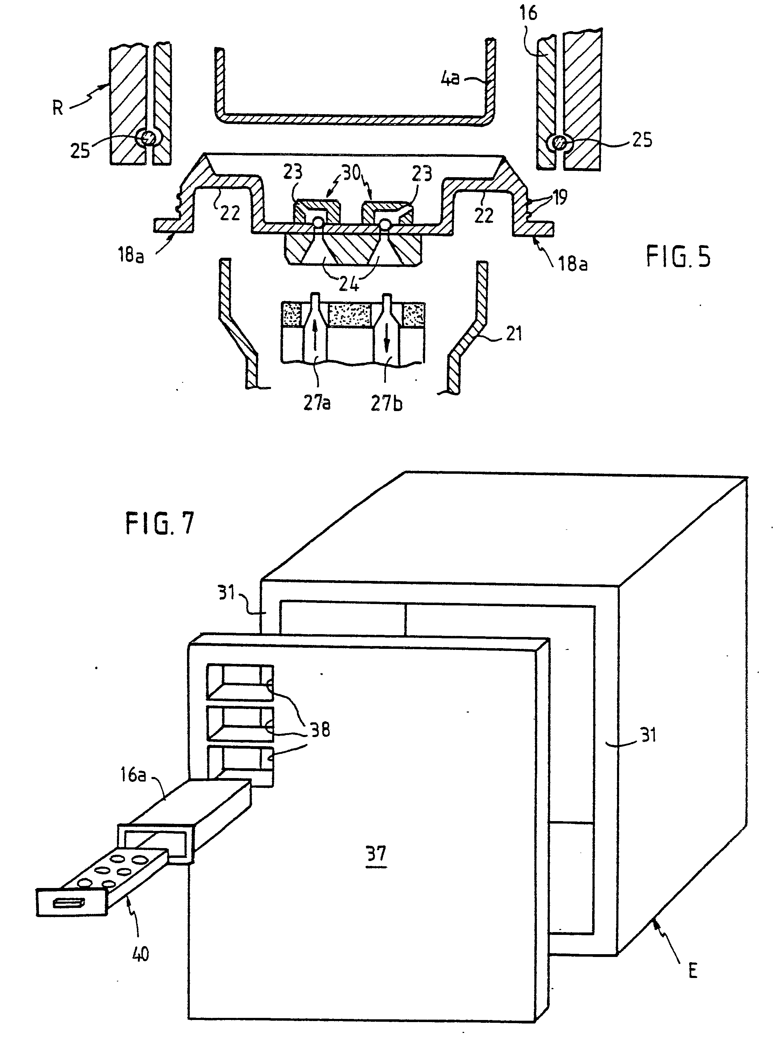

Si le réglage du pH n'est pas nécessaire, par exemple en raison de l'utilisation de milieu tamponné, le couvercle du compartiment est un simple bouchon 18. Dans le cas contraire (qui est actuellement le cas général en culture eucaryote) le couvercle est de type spécial (cf. la référence 18a de la figure 5) pour permettre le recyclage du pH qui est obtenu par une injection d'un mélange air + $CO_2$ (à quelques % de $CO_2$). Dans ce cas (cf. la figure 5), la pince 21 du bras manipulateur B a une course de déplacement qui lui permet de saisir aussi bien les récipients de culture 4a que les couvercles 18a, ces derniers étant évidés et présentant des encoches frontales 22 (cf. la figure 5). Dans la figure 5 les couvercles 18a sont pourvus d'un système à deux clapets 30 dont les orifices externes 24 sont coniques, les orifices internes 23 étant orientés de manière à réaliser un balayage efficace de gaz dans le compartiment, après introduction de l'injecteur et de l'aspirateur 27a, 27b, solidaires de la pince 21, dans les orifices externes 24. En bout de course les clapets 30 sont ouverts.

Suivant le programme et le résultat de la mesure, le $CO_2$ est ajouté seul ou avec tout autre gaz nécessaire (vapeur d'eau, azote, etc...) à partir d'un dispositif d'alimentation filtré en gaz (l'injection et l'aspiration étant commandées par un micro-ordinateur qui pilote d'ailleurs également toutes les opérations suivant le protocole d'expériences classiques prédéfinies). Ainsi, sans ouvrir les couvercles, il est possible de contrôler et réguler autant de fois que nécessaire chacune des boîtes, le cycle complet qui comprend la connexion, l'aspiration, la mesure et l'injection, prenant seulement quelques secondes. Par ailleurs, le volume du compartiment constitue une réserve gazeuse suffisante, vis-à-vis de la consommation cellulaire, pour limiter les besoins à quelques cycles par jour pour chacun des compartiments. Lorsque le récipient de culture 4a doit être extrait du compartiment 16, par exemple en vue d'un changement de milieu, la pince 21 est amenée par le bras manipulateur B selon une position X-Y-Z définie par le programme. La pince 21 est engagée dans le couvercle 18a, puis serrée sur les faces latérales desdites encoches frontales 22. Par un mouvement de retrait de la pince 21 le couvercle 18a est retiré, puis placé dans un logement vide du dispositif de stockage spécialement réservé à cet usage. La pince 21 revient en position et pénètre dans le compartiment 16, saisit la boîte multitrous 4a (qui, rappelons-le, n'a ni couvercle ni bouchon et qui peut être remplacée, soit par une bouteille, soit par des boîtes de Petri), exerce un mouvement de retrait et place le récipient 4a sur le poste de travail correspondant. Après l'exécution de l'opération, le cycle se déroule à l'inverse et se termine par une injection de gaz particulier destiné à chasser le gaz de l'enceinte qui s'est introduit par suite de l'ouverture du compartiment. Les compartiments 16 sont fixés rigidement par une baguette anti-retour 25, qui s'interpose entre la paroi du dispositif de stockage R et la paroi de chaque compartiment 16 en coulissant dans des rainures ménagées dans ces parois (cf. toujours la figure 5).

Lesdits rayonnages R s'accrochent sur des barres verticales 26 (cf. la figure 3) qui sont pourvues de tétons 27 pénétrant dans des logements correspondants 28 ménagés dans des ailettes latérales 29 faisant saillie des côtés verticaux postérieurs de chaque rayonnage. La fixation se fait à l'aide d'un système d'accrochage rapide classique, type Zeus par exemple. Lorsque le dispositif de stockage 1 est réalisé par collage des compartiments 16 entre eux, une plaque perforée est collée sur la face arrière de la boîte ouverte R.

Pour obtenir une bonne assise des dispositifs de stockage R, ceux-ci disposent de pattes inférieures (non représentées) et éventuellement de pattes supérieures si leur taille le justifie.

Précisons en passant que, étant donné que les récipients contenant les cellules peuvent être constitués non seulement par des boîtes multi-

trous 4a ou des bouteilles, mais également par des boîtes de Petri, il va de soi que dans ce dernier cas un support spécial doit être employé : de toutes façons, dans chaque cas l'évaporation est très faible dans le compartiment étanche 16.

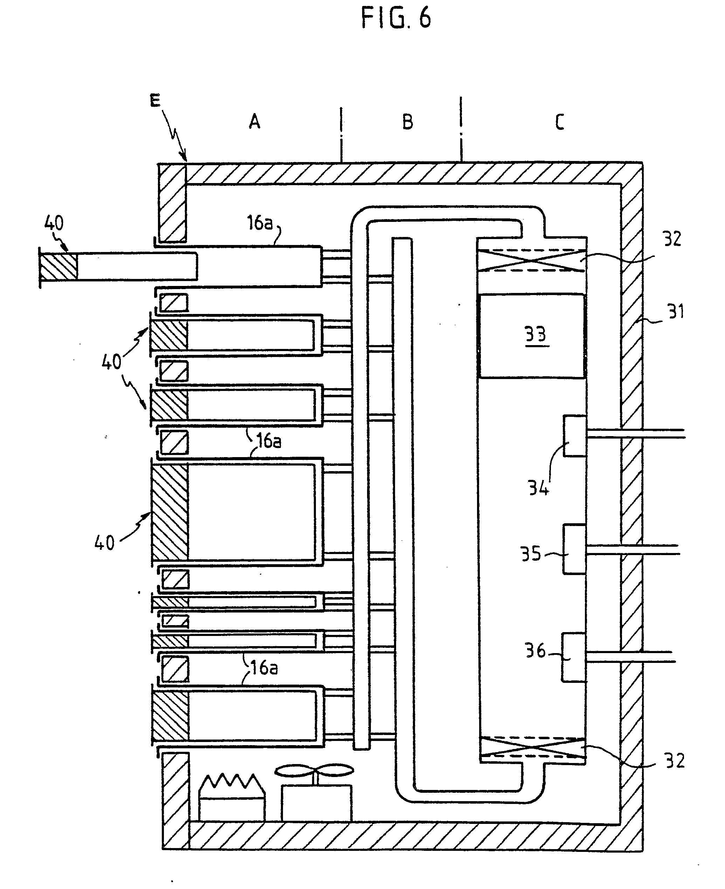

Une forme modifiée de dispositif de stockage est constituée par une étuve étanche E à compartiments 16a schématisée sur la figure 6 et comprenant trois zones principales :

une zone A en face avant, qui rassemble les récipients de culture et qui peut être avantageusement modulaire pour tenir compte de formats variés de récipients de culture ;

une zone intermédiaire B rassemblant les raccordements des compartiments 16a aux différentes alimentations, et

une zone C, disposée à la partie arrière, comprenant le générateur de gaz, les dispositifs de régulation et les filtres.

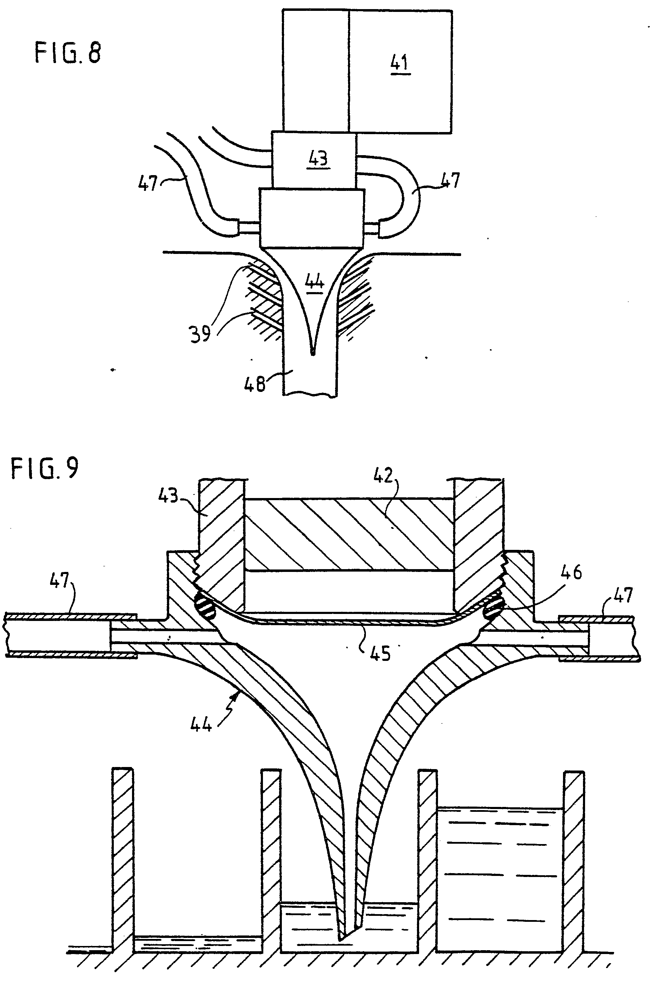

Dans cette zone C, à l'intérieur des parois isothermes 31 et thermostatées, on distingue un ensemble de filtres 32, une pompe destinée à forcer la circulation par l'intermédiaire d'un piston 33 coulissant dans une chambre de mélange où l'on règle la teneur en air, en $CO_2$ et en eau à l'aide de dispositifs classiques, schématiquement représentés, 34, 35, 36. Les compartiments 16a sont connectés à deux groupes de tuyaux destinés à l'alimentation et à l'évacuation, respectivement. Les compartiments 16a, représentés sur la figure 7, sont en métal ou en matière plastique moulée et sont fixés par collage sur le panneau avant isotherme 37 qui a été évidé par matriçages 38 selon des dimensions adaptées aux récipients de culture. Le panneau avant 37 est lui-même collé de façon étanche sur le pourtour de la face avant défini par coopération des parois de l'étuve E.

Les compartiments 16a présentent, par rapport aux compartiments 16 décrits précédemment et représentés sur la figure 5, un rebord sur le pourtour antérieur pour être eux aussi collés, notamment sur le panneau 37, et peuvent contenir des boîtes multitrous classiques : dans ce cas les couvercles sont également identiques à ceux décrits plus haut. On peut avantageusement utiliser des boîtes spéciales telles que représentées sur la figure 7 qui combinent un couvercle étanche et isotherme et qui se présente sous forme de boîtes à tiroirs 40. Chaque boîte spéciale 40 est alors introduite à la main, ou par le bras manipulateur, dans le compartiment 16a et l'étanchéité est assurée également par un joint torique.

L'alimentation en gaz peut se faire par la face avant et avec l'utilisation du bras manipulateur, comme décrit précédemment. En alimentation continue de gaz, l'ouverture et la fermeture des circuits de gaz sont déclenchées par l'enfoncement de la boîte 40 et inversement, par exemple selon un dispositif à bille qui assure également la fermeture mécanique.

L'un des buts principaux de l'invention étant d'automatiser les opérations de prélèvement et d'injection de milieux liquides ou encore de cellules, il existe des dispositifs commerciaux qui peuvent être valablement utilisés, en particulier ceux qui font appel à des cônes jetables. Le choix entre un dispositif réutilisable ou un autre faisant appel à des composants consommables dépend directement du débit du système.

Avec un nombre de récipients de culture élevé, par exemple de l'ordre de 120 boîtes à 96 trous ou logettes chacune, on peut consommer plusieurs milliers de cônes par jour. Le dispositif réutilisable peut traiter sans difficulté cet ensemble de plus de 10 000 logettes dans la journée à raison d'un cycle moyen de 6 secondes comprenant l'aspiration du milieu appauvri, la vidange, le rinçage, la stérilisation et le remplissage en milieu neuf. Cependant, si la cadence l'exige, il est possible de disposer en parallèle plusieurs dispositifs d'aspiration. Le dispositif d'aspiration proposé et représenté aux figures 8 et 9, se rapproche d'un système à piston. Il comprend un micro-moteur pas-à-pas 41 actionnant, par l'intermédiaire d'un système mécanique réducteur, un piston 42 dans un cylindre 43. Un cône d'aspiration 44 en métal, acier inoxydable, aluminium, en verre ou même en plastique (Téflon), se visse sur le cylindre 43. Une membrane 45 est serrée entre le cône 44 et l'embase du cylindre 43. Un joint torique en métal ou matière plastique 46 facilite le montage de la membrane 45, tout en assurant l'étanchéité nécessaire.

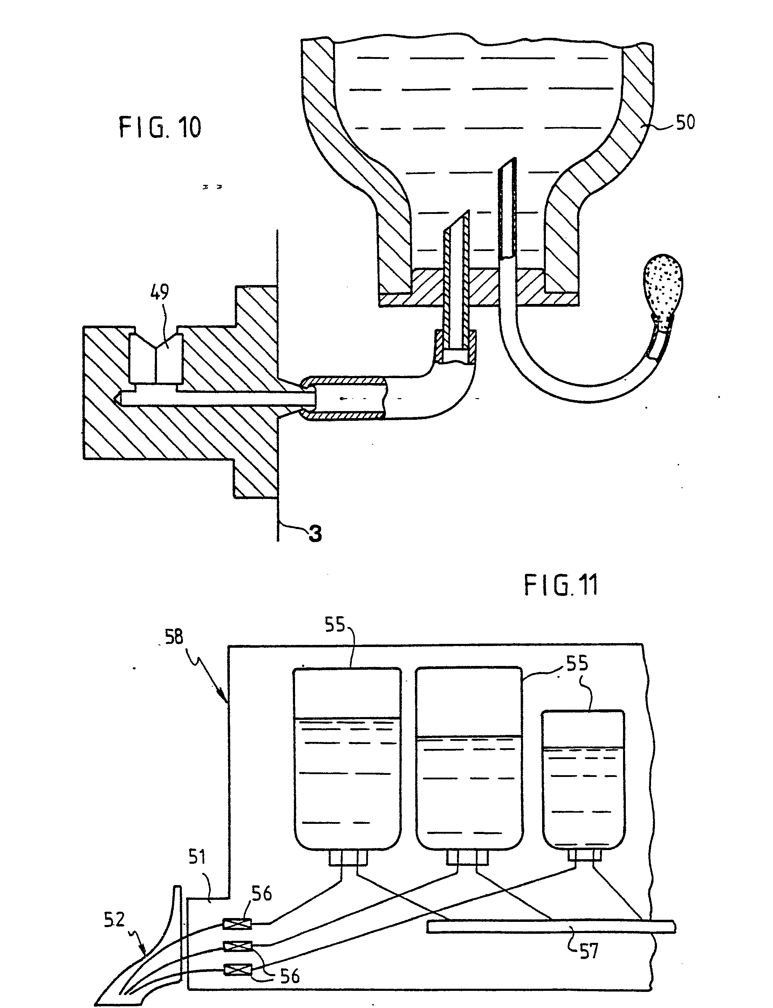

L'extrémité du cône 44 est effilée et en biseau, son diamètre extrême est, par exemple, de l'ordre de 2 mm. Deux conduits 47 permettent l'arrivée de fluides de rinçage (eau stérile) et de stérilisation (vapeur d'eau à haute température, formaline, alcool, etc...). L'évacuation se fait dans un puits 48, ménagé dans la table de travail de l'enceinte, qui sert également de reposoir pour le dispositif mais aussi permet le rinçage externe, la stérilisation et le séchage par des couronnes de trous 39 inclinés dans le sens de l'évacuation. En fonctionnement le bras manipulateur dépose préalablement le récipient de culture sur le poste de travail. Dans le cas le plus simple, ce dernier n'est équipé que de simples butées (non représentées) de calage du récipient destinées à placer tous les récipients dans la même position au millimètre près. Dans ce cas, le bras manipulateur assure à la fois le placement de la boîte, les déplacements du dispositif d'aspiration et du dispositif d'injection, si ceux-ci sont séparés. Notons à ce sujet que le dispositif d'aspiration peut également servir à l'injection comme cela est représenté sur la figure 10. Le dispositif permet de raccorder le cône 44 à une alimentation en liquide par l'intermédiaire d'un raccord 49 auto-obturant en matière souple, notamment en élastomère. Le cône 44, en pénétrant dans ce raccord auto-obturant, écarte les parois internes initialement accolées du matériau souple et l'extrémité du cône entre en contact avec le liquide à aspirer. Le dispositif de figure 10 comprend également un bouchon isotherme qui est raccordé au réservoir 50 placé en ambiance froide 4 °C et se trouve placé à l'extérieur de l'enceinte représentée à la figure 1, tandis que le raccord 49 est soumis au flux laminaire stérile existant à l'intérieur de cette enceinte, en sorte que la contamination ne peut se produire sur les traces résiduelles du milieu nutritif.

Le cycle complet d'un changement de milieu est le suivant. Le récipient déposé et centré n'a pas de couvercle (dans le cas d'une organisation très simple de l'enceinte à rayonnage susdite, le couvercle peut exister et doit alors être enlevé par le bras manipulateur). Le bras manipulateur B saisit le dispositif d'injection préalablement rincé (et, si possible, séché par injection d'air ou vapeur sèche) et le place dans la logette du récipient correspondant au programme de l'ordinateur, le moteur 41 exécute et actionne le piston 43 vers le haut ; la membrane 45 est déprimée dans le même sens et transmet l'aspiration au cône 44. Le volume aspiré, de l'ordre du millilitre n'entre jamais en contact avec le piston 43. Le cône 44 est soulevé et replacé dans le puits 48, le piston 43 est actionné vers le bas et chasse le liquide. Une injection d'eau par l'intermédiaire du conduit 47 rince l'intérieur du cône. Un jet d'air ou de vapeur sèche, toujours introduit par l'intermédiaire du conduit 47, stérilise l'intérieur de ce cône 44 dont la partie extérieure est également soumise simultanément au même traitement par l'intermédiaire des orifices du puits 48.

Comme déjà dit, l'injection de milieu neuf peut se faire par le même dispositif d'aspiration et d'injection des figures 8 et 9 en se connectant sur le dispositif d'alimentation représenté sur la figure 10.

On comprend aisément que de cette manière on peut injecter différents liquides, ce qui rend ce système très modulaire.

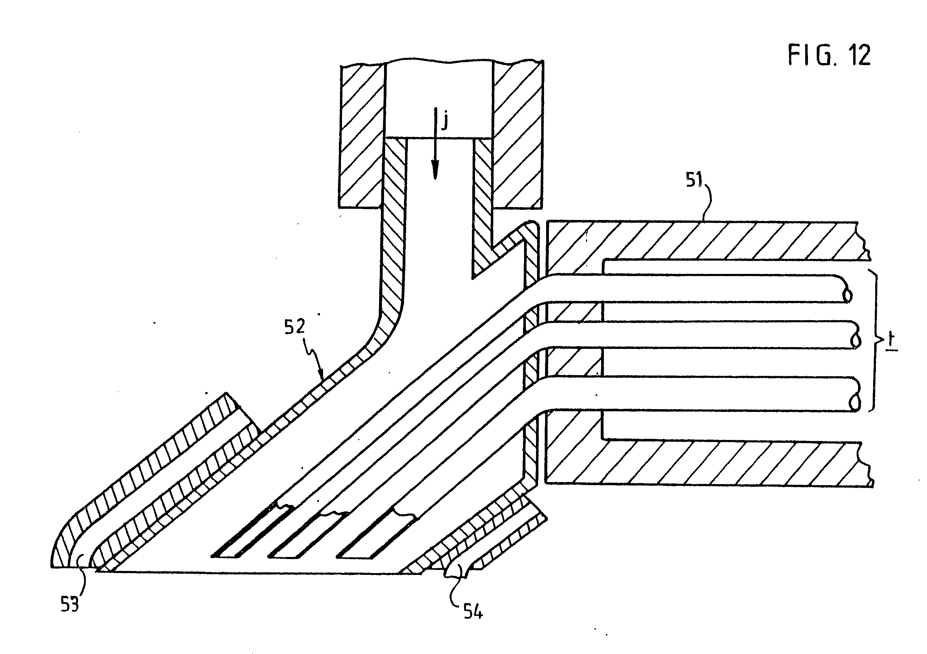

Dans d'autres cas, il est plus intéressant de dissocier la fonction d'aspiration et d'injection, en particulier pour gagner du temps et réduire les risques de dissémination de contamination qui pourraient être créés par l'emploi d'un dispositif unique. A cet effet, les figures 11 et 12 représentent un dispositif d'injection comprenant une arrivée de liquides acheminés par un ou plusieurs tuyaux métalliques t, par exemple six, gainés par un conduit isotherme 51. En 52 on représente une gaine de protection anti-contamination qui achemine un jet d'air j permanent et à faible pression qui empêche le reflux de particules apportées au contact des tuyaux par des remous éventuels. L'inclinaison donnée aux tuyaux t permet de diriger le flux contre les parois des logettes et évite l'impact direct contre les cellules adhérentes du fond de chaque logette. Des fibres optiques 53 et 54, dirigées vers la surface, permettent de repérer la montée du niveau par un procédé sans contact.

La mise en rapport des logettes préalablement vidées et de ce dispositif d'injection peut se faire, soit par le bras manipulateur B, qui transporte le dispositif d'injection rendu mobile, soit par déplacement de la boîte sous ce dispositif rendu fixe. Il est également aisé de coupler le dispositif d'aspiration des figures 8 et 9 au dispositif d'injection des figures 11 et 12. Les flacons 55 contenant les produits à injecter peuvent être reliés à des systèmes de vannes stérilisables 56 fonctionnant en tout ou rien, par exemple par écrasement des tuyaux d'alimentation en élastomère. Une arrivée 57 d'air stérile à basse pression met, de façon connue en soi, les flacons 55 en pression à l'intérieur de l'enceinte réfrigérée 58. En fonctionnement, le récipient de culture déplacé par le bras manipulateur B met une logette déterminée sous le dispositif d'injection, le clapet ou les clapets 56 correspondant au programme s'ouvrent, la logette se remplit et, dès que le niveau fixé est atteint, le clapet ou les clapets 56 sont refermés.

## Revendications

1. Appareil modulaire de culture cellulaire comprenant, dans une même enceinte étanche (2) et à parois isothermes (3), en combinaison :
   au moins un dispositif de stockage (1, R, E) de récipients de culture, notamment constitués par des boîtes multitrous ou des flacons,
   un dispositif de régulation de température (7),
   un dispositif de stérilisation (11, 12, 13, 14) par flux laminaire filtré, en circuit fermé,
   un dispositif d'alimentation et de régulation de gaz, tels que de l'air, du $CO_2$ et de la vapeur,
   au moins un dispositif permettant d'effectuer une opération appropriée sur les cultures cellulaires,
   lequel appareil est caractérisé en ce que ledit dispositif de stockage de récipients de culture est un dispositif modulaire qui comporte une pluralité de modules constituant des rayonnages (R) pourvus de glissières (17), le long desquelles coulissent des compartiments (16) étanches contenant des récipients de culture (4a) et obturés par des couvercles, notamment légèrement coniques (18, 18a), qui sont équipés d'une poignée et de joints toriques d'étanchéité.

2. Appareil selon la revendication 1, caractérisé en ce que ladite enceinte étanche (2) contient également au moins un bras (B) automatique et programmable, qui est pourvu d'une pince de manipulation (21) qui effectue toutes les opérations correspondant à l'emplacement de chaque dispositif permettant d'effectuer une opération appropriée sur les cultures cellulaires et définissant un poste de travail modulaire choisi en fonction des besoins, et un micro-ordinateur de pilotage de toutes les opérations suivant un protocole d'expériences classiques, ainsi que spécialement définies en fonction des besoins, lequel micro-ordinateur assure également le traitement des données.

3. Appareil selon la revendication 1, caractérisé en ce que chaque compartiment (16) est tenu en place par l'intermédiaire d'une baguette anti-retour (25) s'interposant entre la paroi du module de rayonnage (R) et celle du compartiment (16) lui-même.

4. Appareil selon la revendication 1, caractérisé en ce que, la liaison entre modules de rayonnage (R) contigus se fait par l'intermédiaire de barres verticales (26), notamment en forme de T, qui sont pourvues de tétons d'accrochage (27) pénétrant dans des logements correspondants (28) ménagés dans des ailettes latérales (29) faisant saillie à partir des côtés verticaux postérieurs de chaque module de rayonnage (R).

5. Appareil selon la revendication 2, caractérisé en ce que, chaque compartiment (16) est transparent et un espace approprié est ménagé au-dessus et au-dessous de chaque compartiment de manière à introduire, à l'aide dudit bras manipulateur (B), un dispositif optique permettant d'effectuer des mesures appropriées, notamment de colorimétrie et d'opacité.

6. Appareil selon la revendication 2, caractérisé en ce que l'alimentation en gaz de chaque compartiment se fait automatiquement à l'aide du bras manipulateur (B), qui aspire le gaz à renouveler, ce qui permet d'en analyser la composition, et qui injecte le gaz de renouvellement, à travers un système à deux clapets (30) ménagés dans la poignée (20) du couvercle d'obturation (18a) de chaque compartiment (16) et pourvus de deux conduits communiquant avec un conduit d'injection (27a) et un conduit d'aspiration (27b), respectivement, portés par le bras manipulateur (B) et activés lors de la préhension de la poignée (20) du couvercle (18a) par la pince (21) du bras (B).

7. Appareil modulaire de culture cellulaire selon la revendication 1, modifié en ce que le dispositif de stockage modulaire est caractérisé par une pluralité de modules constituant des étuves (E) à parois isothermes et à compartiments étanches (16a) et comprenant trois zones principales (A, B, C), à savoir :

une première zone (A), disposée à la partie avant, rassemblant les récipients de cultures dans lesdits compartiments (16a),

une deuxième zone (B), disposée à la partie médiane, rassemblant les raccordements entre chaque compartiment (16a) et les différentes alimentations en gaz, et

une troisième zone (C), disposée à la partie arrière, rassemblant les dispositifs d'alimentation en gaz, les dispositifs de régulation et les filtres, lesquels compartiments (16a) sont pourvus d'un rebord sur leur face avant et sont appliqués, notamment par collage, sur une plaque (37) pourvue d'ouvertures (38) et fixée elle aussi — notamment par collage — sur le pourtour de la face avant de chaque étuve (E), lesdits compartiments étant en outre disposés suivant des rangées verticales et ayant des dimensions variées.

8. Appareil selon l'une quelconque des revendications 2 et 7, caractérisé en ce que l'alimentation en gaz de chaque compartiment (16a) se fait automatiquement à l'aide d'ergots à billes permettant l'ouverture des vannes d'alimentation lors de la fermeture des compartiments (16a), tandis que l'ouverture de ces derniers désactive les vannes d'alimentation, et en ce que les récipients de cultures, notamment du type multitrous, sont rendus solidaires des couvercles des compartiments (16a), qui constituent ainsi des boîtes spéciales à tiroir (40) destinées à coopérer avec lesdits ergots d'activation de l'alimentation en gaz au moment de l'introduction de ces boîtes spéciales (40) dans lesdits compartiments (16a).

9. Appareil selon la revendication 7, caractérisé en ce que, lesdits dispositifs d'alimentation en gaz concourent dans une chambre de mélange où l'on règle la teneur en air, en $CO_2$ et en vapeur d'eau, notamment, à l'aide des dispositifs de réglage (34, 35, 36) connus en eux-mêmes, chambre dans laquelle coulisse un piston (33) dirigeant le mélange de gaz vers le ou les compartiments (16a) sélectionnés.

10. Appareil selon l'une quelconque des revendications 1 à 7, caractérisé en ce que, lesdits postes de travail modulaires sont constitués par au moins l'un des dispositifs suivants :

un dispositif d'alimentation en milieu nutritif enrichi et d'évacuation du milieu appauvri,

et/ou un dispositif de rinçage et de stérilisation des récipients,

et/ou un dispositif de distribution de milieux nutritifs,

et/ou un dispositif de distribution de substances, notamment pharmacologiques,

et/ou un dispositif de distribution de cellules,

et/ou au moins un dispositif d'observation comprenant des optiques photoniques, notamment acoustiques, couplées ou non à des platines motorisées, ainsi qu'à un analyseur d'images, à un appareil photographique ou à un magnétoscope,

et/ou des dispositifs d'analyse physico-chimique,

et/ou des dispositifs pour repiquage,

et/ou des dispositifs de stockage variés,

et/ou des dispositifs de préparation de récipients de culture, notamment par pulvérisation, de substrats appropriés, tels que du collagène.

11. Appareil selon l'une quelconque des revendications 1 à 10, caractérisé en ce que, ledit dispositif d'évacuation du milieu nutritif appauvri consiste en un dispositif d'aspiration comprenant un micro-moteur pas-à-pas (41) qui actionne, par l'intermédiaire d'un système mécanique réducteur, un piston (42) dans un cylindre (43) fileté à sa base inférieure, autour de laquelle se visse un cône d'aspiration (44) à extrémité effilée et biseautée, une membrane (45) étant serrée entre ce cône (44) et l'embase dudit cylindre (43) et supportée par un joint torique (46) qui assure en même temps l'étanchéité nécessaire.

12. Appareil selon la revendication 11, caractérisé en ce que, le cône (44) est relié à deux conduits (47) permettant l'arrivée de fluides de rinçage et de stérilisation de la partie interne du cône (44) après l'opération d'aspiration et d'évacuation du milieu appauvri.

13. Appareil selon la revendication 12, caractérisé en ce que, l'évacuation du milieu aspiré se fait dans un puits (48) ménagé dans la table de travail (6) de ladite enceinte, lequel puits (48) sert également de reposoir pour le dispositif d'aspiration et est pourvu d'une série de couronnes de conduits (39), décalés et inclinés dans le sens de l'évacuation, qui sont destinés au rinçage externe, à la stérilisation et au séchage.

14. Appareil selon la revendication 11, caractérisé en ce que, ledit dispositif d'aspiration coopère avec un dispositif d'alimentation en liquide pourvu d'un raccord auto-obturant (49) en matière souple, notamment en élastomère, qui est soumis au flux laminaire existant à l'intérieur de l'enceinte et dans lequel est introduit ledit cône d'aspiration (44), tandis que le réservoir d'alimentation (50) est disposé à l'extérieur de l'enceinte et en ambiance froide, l'injection de milieu nutritif liquide neuf ou enrichi se faisant par inversion du sens de rotation dudit moteur pas-à-pas (41).

15. Appareil selon la revendication 11, caractérisé en ce que, l'injection de milieu nutritif enrichi se fait à l'aide d'un dispositif d'injection qui est indépendant dudit dispositif d'aspiration et qui comprend des flacons (55) contenant les liquides à injecter à l'intérieur d'un bloc réfrigérant (58), ces liquides étant acheminés par un ou plusieurs tuyaux (t), notamment en élastomère, réunis en faisceaux et enveloppés par une gaine isotherme (51) faisant saillie de la base du bloc réfrigérant (58), le débit de chaque tuyau (t) d'alimentation étant contrôlé par une vanne stérilisable (56) fonctionnant en tout ou rien.

16. Appareil selon la revendication 15, caractérisé en ce que, la partie terminale du faisceau de tuyaux (t) est pourvue d'une gaine de protection anti-contamination (52) dans laquelle est acheminé un jet d'air (j) permanent et à faible pression qui empêche le reflux des particules de liquide, cette gaine anti-contamination (52) et les extrémités desdits tuyaux d'alimentation (t) étant inclinés de façon à diriger le liquide d'injection contre la paroi desdites logettes de culture et à éviter l'impact direct contre les cellules qui adhèrent au fond de chaque logette.

17. Appareil selon l'une quelconque des revendications 1 à 16, caractérisé en ce qu'il comporte également un dispositif de recueil des liquides versés accidentellement sur un poste de travail, qui est constitué de préférence par un entonnoir unique placé au-dessous de la table de travail et qui est pourvu d'un orifice latéral de communication avec le circuit de stérilisation par flux laminaire.

**Claims**

1. Modular cell culture apparatus comprising, in the same tight enclosure (2) with isothermal walls (3), in combination :
at least one storage device (1, R, E) for culture containers constituted in particular by multi-hole boxes or bottles,
a temperature regulating device (7),
an arrangement for sterilization (11, 12, 13, 14) by filtered laminar flow in closed circuit,
a device for supplying and regulating gas, such as air, $CO_2$ and steam,
at least one device enabling a suitable operation to be carried out on the cell cultures,
which apparatus is characterised in that the said storage device for culture containers is a modular device which comprises a plurality of modules constituting shelvings (R) provided with guides (17) along which slide tight compartments (16) containing culture containers (4a) and closed by covers, in particular slightly conical covers (18, 18a), which are equipped with a handle and with toroidal seals.

2. Apparatus according to claim 1, characterised in that the said tight enclosure (2) also contains at least one automatic and programmable arm (B) provided with handling tongs (21) which effect all the operations corresponding to the location of each device enabling a suitable operation to be carried out on the cell cultures and defining a modular working station chosen in accordance with requirements, and a microcomputer for handling all the operations according to a procedure of conventional tests, as well as tests specially defined in accordance with requirements, which microcomputer also ensures the processing of the data.

3. Apparatus according to claim 1, characterised in that each compartment (16) is held in place through the medium of a non-return rod (25) interposed between the wall of the shelving module (R) and that of the compartment (16) itself.

4. Apparatus according to claim 1, characterised in that connection between adjacent shelving modules (R) is effected through the medium of vertical bars (26), in particular of T-shape, which are provided with anchoring studs (27) entering into corresponding seats (28) formed in lateral flanges (29) projecting from the rear vertical sides of each shelving module (R).

5. Apparatus according to claim 2, characterised in that each compartment (16) is transparent and a suitable space is arranged above and below each compartment so as to introduce, with the aid of the said handling arm (B), an optical device enabling suitable measurements, in particular colorimetry and opacity measurements, to be carried out.

6. Apparatus according to claim 2, characterised in that the supply of each compartment with gas is effected automatically with the aid of the handling arm (B), which sucks up the gas to be renewed, enabling the composition thereof to be analyzed, and injects the renewal gas, through a system having two clack valves (30) arranged in the handle (20) of the closing cover (18a) of each compartment (16) and provided with two ducts communicating with an injection duct (27a) and a suction duct (27b), respectively, which are borne by the handling arm (B) and activated on the gripping of the handle (20) of the cover (18a) by the tongs (21) of the arm (B).

7. Modular cell culture apparatus according to claim 1, modified in that the modular storage device is characterised by a plurality of modules constituting cupboards (E) with isothermal walls and tight compartments (16a) and comprising three main zones (A, B, C), namely :

a first zone (A) disposed at the front and grouping together the culture containers in the said compartments (16a),

a second zone (B) disposed in the middle and grouping together the connections between each compartment (16a) and the various gas supplies, and

a third zone (C) disposed at the rear and grouping together the gas supply devices, the regulating devices and the filters, which compartments (16a) are provided with a flange on their front face and are applied, in particular by cementing, to a plate (37) provided with openings (38) and also fixed, in particular by cementing, to the periphery of the front face of each cupboard (E), the said compartments being moreover arranged in vertical rows and having varied dimensions.

8. Apparatus according to any one of claims 2 and 7, characterised in that the supply of each compartment (16a) with gas is effected automatically with the aid of ball catches permitting the opening of the supply valves on the closing of the compartments (16a), while the opening of the latter deactivates the supply valves, and the culture containers, in particular of the multi-hole type, are rendered fast with the covers of the compartments (16a), which thus constitute special drawer-type boxes (40) intended to cooperate with the said catches for activating the supply of gas at the instant of introduction of these special boxes (40) into the said compartments (16a).

9. Apparatus according to claim 7, characterised in that the said gas supply devices converge in a mixing chamber where the content of air, $CO_2$ and steam is regulated, in particular with the aid of the regulating devices (34, 35, 36) known per se, in which chamber there slides a piston (33) directing the mixture of gases towards the selected compartment or compartments (16a).

10. Apparatus according to any one of claims 1 to 7, characterised in that the said modular working stations are constituted by at least one of the following devices :

a device for supplying enriched nutrient medium and removing the impoverished medium,

and/or a device for rinsing and sterilizing the containers,

and/or a device for distributing nutrient media,

and/or a device for distributing substances, in particular pharmacological substances,

and/or a device for distributing cells,

and/or at least one observation device comprising photonic optics, in particular acoustic, coupled or not to motorized stages, as well as to an image analyzer, a camera or a magnetoscope,

and/or physicochemical analysis devices,

and/or devices for subculture,

and/or various storage devices,

and/or devices for preparing culture containers, in particular by pulverization of suitable substrates, such as collagen.

11. Apparatus according to any one of claims 1 to 10, characterised in that the said device for removing the impoverished nutrient medium consists of a suction device comprising a stepping micromotor (41) which, through the medium of a mechanical reducing system, actuates a piston (42) in a cylinder (43) threaded at its lower base, around which base there is screwed a suction cone (44) with a tapered and chamfered end, a diaphragm (45) being clamped between this cone (44) and the base of the said cylinder (43) and being supported by a toroidal packing (46) which ensures the necessary tightness at the same time.

12. Apparatus according to claim 11, characterised in that the cone (44) is connected to two tubes (47) permitting the inlet of fluids for rinsing and sterilizing the inside of the cone (44) after the operation of suction and removal of the impoverished medium.

13. Apparatus according to claim 12, characterised in that the removal of the medium sucked up is effected into a well (48) provided in the working table (6) of the said enclosure, which well (48) also serves as a resting place for the suction device and is provided with a series of rings of ducts (39) which are off-set and inclined in the direction of removal and are intended for external rinsing, for sterilization and for drying.

14. Apparatus according to claim 11, characterised in that the said suction device cooperates with a liquid supply device provided with a self-sealing connector (49) of flexible material, in particular elastomer, which is subjected to the laminar flow existing inside the enclosure and into which the said suction cone (44) is introduced, while the supply reservoir (50) is disposed outside the enclosure and in a cold environment, the injection of fresh or enriched liquid nutrient medium being effected by reversal of the direction of rotation of the said stepping motor (41).

15. Apparatus according to claim 11, characterised in that the injection of enriched nutrient medium is effected with the aid of an injection device which is independent of the said suction device and comprises bottles (55) containing the liquids to be injected inside a refrigerating block (58), these liquids being conveyed by one or more tubes (t), in particular of elastomer, brought together in a group and surrounded by an isothermal casing (51) projecting from the base of the refrigerating block (58), the rate of flow from each supply tube (t) being controlled by a sterilizable valve (56) operating fully or not at all.

16. Apparatus according to claim 15, characterised in that the terminal portion of the group of tubes (t) is provided with a protective anti-contamination sheath (52) into which is conveyed a permanent and low-pressure air jet (j) which prevents return flow of the particles of liquid, this anti-contamination sheath (52) and the ends of the said supply tubes (t) being inclined so as to direct the injection liquid against the wall of the said culture receptacles and avoid direct impact against the cells adhering to the bottom of each receptacle.

17. Apparatus according to any one of claims 1 to 16, characterised in that it also comprises a device for collecting liquids spilled accidentally on a working station, which is preferably constituted by a single funnel located below the working table and provided with a lateral orifice communicating with the laminar flux sterilizing circuit.

**Patentansprüche**

1. Modulare Einrichtung für Zellkultur, umfassend in einem gleichen dichten und mit isothermen Wänden (3) versehenen umschlossenen Raum (2) in Kombination :
wenigstens eine Speicherungseinrichtung (1, R, E) für Kulturbehälter, die insbesondere von Mehrlochkästen oder Fläschchen gebildet sind,
eine Einrichtung zum Regeln der Temperatur (7),
eine Einrichtung zur Sterilisation (11, 12, 13, 14) durch laminaren filtrierten Fluß in geschlossenem Kreislauf,
eine Einrichtung zur Versorgung mit und zur Regelung von Gas, wie von Luft, von $CO_2$ und von Dampf,
wenigstens eine Einrichtung, welche es gestattet, eine geeignete Einwirkung auf die Zellkulturen zu bewerkstelligen,
welche Einrichtung dadurch gekennzeichnet ist, daß die Speicherungseinrichtung für Kulturbehälter eine modulare Einrichtung ist, die eine Mehrzahl von Modulen umfaßt, die Etagengestelle (R) bilden, welche mit Gleitschienen (17) versehen sind, längs denen dichte Fächer (16) gleiten, die Kulturbehälter (4a) enthalten und durch, insbesondere leicht konische (18, 18a) Abdeckungen verschlossen sind, die mit einem Griff und mit ringförmigen Dichtungen zur Abdichtung versehen sind.

2. Einrichtung nach Anspruch 1, dadurch gekennzeichnet, daß der dichte umschlossene Raum (2) weiterhin wenigstens einen automatischen und programmierbaren Arm (B) enthält, der mit einer Manipulationszange (21) versehen ist, die alle Tätigkeiten durchführt, welche dem Standort jeder Einrichtung entsprechen, die es gestattet, eine geeignete Einwirkung auf die Zellkulturen auszuführen und einen modularen Arbeitsplatz festlegt, der in Abhängigkeit von den Bedürfnissen gewählt ist, und eine Mikrodatenverarbeitungsanlage zum Steuern von allen Tätigkeiten gemäß einem Protokoll von klassischen, sowie speziell in Abhängigkeit von den Bedürfnissen bestimmten Versuchen, welche Mikrodatenverarbeitungsanlage ferner die Verarbeitung der Daten sicherstellt.

3. Einrichtung nach Anspruch 1, dadurch gekennzeichnet, daß jedes Fach (16) mittels eines Rücklaufsicherungsstabs (25), der sich zwischen die Wand des Etagengestellmoduls (R) und diejenige des Fachs (16) selbst zwischenfügt, an Ort und Stelle gehalten wird.

4. Einrichtung nach Anspruch 1, dadurch gekennzeichnet, daß die Verbindung zwischen benachbarten Etagengestellmodulen (R) mittels vertikaler Stangen (26), insbesondere in T-Form, erfolgt, die mit Verankerungszapfen (27) versehen sind, welche in entsprechende Sitze (28) eindringen, die in seitlichen Stegen (29) angeordnet sind, welche von den vertikalen hinteren Seiten jedes Etagengestellmoduls (R) vorstehen.

5. Einrichtung nach Anspruch 2, dadurch gekennzeichnet, daß jedes Fach (16) transparent ist und ein geeigneter Raum über und unter jedem Fach in der Weise ausgespart ist, daß mit Hilfe des Manipulatorarms (B) eine optische Einrichtung einführbar ist, welche es gestattet, geeignete Messungen, insbesondere der Kolorimetrie und der opazität, durchzuführen.

6. Einrichtung nach Anspruch 2, dadurch gekennzeichnet, daß die Gasversorgung jedes Fachs automatisch mit Hilfe des Manipulatorarms (B) erfolgt, der das zu erneuernde Gas ansaugt, was es ermöglicht, seine Zusammensetzung zu analysieren, und der das Gas für die Erneuerung injiziert, und zwar über ein System mit zwei Sperrventilen (30), die in dem Griff (20) der Abdeckung (18a) für das Verschließen jedes Fachs (16) vorgesehen und mit zwei Leitungen versehen sind, die mit einer Injektionsleitung (27a) bzw. einer Ansaugleitung (27b) kommunizieren, welche von dem Manipulatorarm (B) getragen und während des Greifens des Griffs (20) der Abdeckung (18a) durch die Zange (21) des Arms (B) aktiviert werden.

7. Modulare Einrichtung für Zellkultur nach Anspruch 1, die dahingehend abgewandelt ist, daß die modulare Speicherungseinrichtung gekennzeichnet ist durch eine Mehrzahl von Modulen, welche Wärmeschränke (E) mit isothermen Wänden und mit dichten Fächern (16a) bilden und drei Hauptzonen (A, B, C) umfassen, nämlich :
eine erste, in dem vorderen Teil vorgesehene Zone (A), welche die Kulturbehälter in den erwähnten Fächern aufnimmt,
eine zweite, in dem mittleren Teil vorgesehene Zone (B), welche die Verbindungen zwischen jedem Fach (16a) und den unterschiedlichen Gasversorgungen aufnimmt, und
eine dritte, im hinteren Teil vorgesehene Zone (C), welche die Gasversorgungseinrichtungen, die Regeleinrichtungen und die Filter aufnimmt, welche Fächer (16a) mit einer Randleiste auf ihrer Vorderseite versehen und, insbesondere durch Kleben, auf einer Platte (37) angebracht sind, die mit Öffnungen (38) versehen ist und die auch — insbesondere durch Kleben — auf dem Umfang der Vorderseite jedes Wärmeschranks (E) befestigt ist, wobei diese Fächer außerdem gemäß vertikalen Reihen angeordnet sind und variierende Dimensionen haben.

8. Einrichtung nach irgendeinem der Ansprüche 2 und 7, dadurch gekennzeichnet, daß die Gasversorgung jedes Fachs (16a) automatisch mit Hilfe von Kugelvorsprüngen erfolgt, welche die Öffnung der Versorgungsventile während des Schließens der Fächer (16a) gestatten, während die Öffnung der letzteren die Versorgungsventile deaktiviert, und daß die Kulturbehälter, insbesondere vom Mehrlochtyp, mit den Abdeckungen der Fächer (16a) fest verbunden sind, welche so Spezialschubkästen (40) bilden, die dazu bestimmt sind, mit den erwähnten Vorsprüngen zur Aktivierung der Gasversorgung im Augenblick des Einführens dieser Spezialkästen (40) in die erwähnten Fächer (16a) zusammenzuwirken.

9. Einrichtung nach Anspruch 7, dadurch gekennzeichnet, daß die erwähnten Gasversorgungseinrichtungen in einer Mischkammer zusammenlaufen, wo der Gehalt an Luft, an $CO_2$ und an Wasserdampf, insbesondere mit Hilfe von an sich bekannten Regeleinrichtungen (34, 35, 36), eingestellt wird, wobei in der Kammer ein Kolben (33) gleitet, der die Gasmischung nach dem oder den gewählten Fächern (16a) lenkt.

10. Einrichtung nach irgendeinem der Ansprüche 1 bis 7, dadurch gekennzeichnet, daß die modularen Arbeitsplätze von wenigstens einer der folgenden Einrichtungen gebildet sind :

eine Einrichtung zur Versorgung mit einem angereicherten Nährmittel und zur Entleerung der verarmten Mittels,

und/oder eine Einrichtung zum Auswaschen und zur Sterilisation von Behältern,

und/oder eine Einrichtung zur Verteilung der Nährmittel,

und/oder eine Einrichtung zur Verteilung von, insbesondere pharmakologischen, Substanzen,

und/oder eine Einrichtung zur Verteilung von Zellen,

und/oder wenigstens eine Einrichtung zur Beobachtung, die Photonoptiken, insbesondere akustische, umfaßt, welche an motorisierte Platinen angekoppelt sind oder nicht, sowie einen Bildanalysator, mit einem fotografischen Apparat oder mit einem Magnetoskop,

und/oder physiko-chemische Analysiereinrichtungen,

und/oder eine Einrichtung zur Versetzung,

und/oder verschiedene Speichereinrichtungen,

und/oder Einrichtungen zur Präparation von Kulturbehältern, insbesondere durch Pulverisierung von geeigneten Substraten, wie Kollagen.

11. Einrichtung nach irgendeinem der Ansprüche 1 bis 10, dadurch gekennzeichnet, daß die erwähnte Einrichtung zum Entleeren des verarmten Nährmittels aus einer Ansaugeinrichtung besteht, die einen Mikroschrittmotor (41) umfaßt, welcher mittels eines mechanischen Untersetzungsgetriebesystems einen Kolben (42) in einem Zylinder (43) betreibt, der an seiner unteren Basis mit einem Gewinde versehen ist, an welchem ein Ansaugkonus (44) mit zugespitztem und abgeschrägtem Ende angeschraubt wird, wobei eine Membrane (45) zwischen dem Konus (44) und der Fußfläche des Zylinders (43) befestigt und durch eine ringförmige Dichtung (46), welche gleichzeitig die notwendige Dichtigkeit sicherstellt, gehalten ist.

12. Einrichtung nach Anspruch 11, dadurch gekennzeichnet, daß der Konus (44) mit zwei Leitungen (47) verbunden ist, welche die Ankunft von Fluiden für das Auswaschen und die Sterilisation des inneren Teils des Konus (44) nach der Tätigkeit des Ansaugens und der Entleerung des verarmten Mittels gestatten.

13. Einrichtung nach Anspruch 12, dadurch gekennzeichnet, daß die Entleerung des angesaugten Mittels in einen Schacht (48) erfolgt, der in dem Arbeitstisch (6) des umschlossenen Raums angebracht ist, welcher Schacht (48) ferner als Ablage für die Ansaugeinrichtung dient und mit einer Reihe von Kränzen von Leitungen (39) versehen ist, die in Entleerungsrichtung gestaffelt und geneigt sind, welche zum externen Auswaschen, zur Sterilisation und zur Trocknung bestimmt sind.

14. Einrichtung nach Anspruch 11, dadurch gekennzeichnet, daß die Ansaugeinrichtung mit einer Flüssigkeitsversorgungseinrichtung zusammenwirkt, die mit einem selbstverschließenden Anschluß (49) aus elastischem Material, insbesondere aus Elastomer, versehen ist, welcher dem laminaren Fluß ausgesetzt ist, der im Inneren des umschlossenen Raums vorhanden ist und in welchen der erwähnte Ansaugkonus (44) eingeführt wird, während das Versorgungsreservoir (50) im Äußeren des umschlossenen Raums und in der kalten Umgebung vorgesehen ist, wobei die Injektion des neuen oder angereicherten flüssigen Nährmittels durch Umkehrung der Drehrichtung des erwähnten Schrittmotors (41) erfolgt.

15. Einrichtung nach Anspruch 11, dadurch gekennzeichnet, daß die Injektion des angereicherten Nährmittels mit Hilfe einer Injektionseinrichtung erfolgt, die unabhängig von der Ansaugeinrichtung ist und die Flaschen (55) umfaßt, welche im Inneren eines Kühlblocks (58) zu injizierende Flüssigkeiten enthalten, wobei diese Flüssigkeiten durch ein oder mehrere Röhren (t), insbesondere aus Elastomer, weitergeleitet werden, die im Bündel vereinigt und von einer isothermen Hülse (51) eingehüllt sind, welche von der Basis des Kühlblocks (58) vorspringt, wobei der Durchsatz jeder Versorgungsröhre (t) durch ein sterilisierbares Ventil (56) kontrolliert wird, das entweder ganz offen oder geschlossen ist.

16. Einrichtung nach Anspruch 15, dadurch gekennzeichnet, daß der Endteil des Bündels von Röhren (t) mit einer Antikontaminationsschutzhülse (52) versehen ist, in welche ein permanenter und einen schwachen Druck aufweisender Luftstrahl weitergeleitet wird, der den Rückfluß von Flüssigkeitsteilchen verhindert, wobei diese Antikontaminationshülse (52) und die Enden der Versorgungsröhren (t) in der Weise geneigt sind, daß sie die Injektionsflüssigkeit gegen die Wand der erwähnten Kulturzellen richten, und daß ein direktes Auftreffen gegen die Zellen, welche am Boden jeder Zelle anhaften, vermieden wird.

17. Einrichtung nach irgendeinem der Ansprüche 1 bis 16, dadurch gekennzeichnet, daß sie weiterhin eine Einrichtung zum Erfassen von Flüssigkeiten enthält, die zufällig auf einem Arbeitsplatz vergossen worden sind, welche vorzugsweise von einem einzigen Trichter gebildet ist, der unterhalb des Arbeitstischs angeordnet ist und der mit einer seitlichen Öffnung zur Verbindung mit dem Sterilisationskreislauf durch laminaren Fluß versehen ist.

FIG.1

0 165 172

FIG. 2

FIG.3

FIG. 4

0 165 172

FIG. 5

FIG. 7

3

# FIG. 6

FIG. 8

<u>41</u>

<u>43</u>

47

47

<u>44</u>

39

48

FIG. 9

43

42

47

46

47

45

44

FIG. 10

50

49

3

FIG. 11

58

55

55

51

52

56

56

57

FIG. 12

0 165 172